# EUROPEAN PATENT SPECIFICATION

(11) **EP 1 863 403 B1**
(45) Date of publication and mention of the grant of the patent: **13.08.2014**
(21) Application number: 06739564.0
(22) Date of filing: 24.03.2006
(51) Int. Cl.: A61F 2/01

(54) **METHODS AND APPARATUSES FOR DISPOSITION OF A MEDICAL DEVICE ONTO AN ELONGATE MEDICAL DEVICE**
VERFAHREN UND VORRICHTUNGEN ZUM ANBRINGEN EINER MEDIZINISCHEN VORRICHTUNG AN EINER LÄNGLICHEN MEDIZINISCHEN VORRICHTUNG
MÉTHODES ET APPAREILLAGES POUR DISPOSER UN APPAREIL MÉDICAL SUR UN APPAREIL MÉDICAL ALLONGÉ

(30) Priority: 29.03.2005 US 92082
(43) Date of publication of application: 12.12.2007
(73) Proprietor: Boston Scientific Limited, Hastings Christ Church (BB)
(72) Inventor: VRBA, Anthony, C., Maple Grove, MN 55311 (US)
(74) Representative: Vossius & Partner
(86) International application number: PCT/US2006/010849
(87) International publication number: WO 2006/104910

(56) References cited:
- US-A1- 2004 039 411
- US-A1- 2004 127 933
- US-A1- 2004 138 693
- US-A1- 2004 199 198
- US-B1- 6 371 971

## Description

The invention pertains to methods and apparatuses that permit a medical practitioner such as a doctor to add functionality such as distal protection to medical devices such as guidewires.

Guidewires are one of the most common devices used in intravascular procedures. They are designed to be easy to navigate to the treatment site and they permit other therapeutic devices to be advanced easily to the treatment. Guidewires with a distal protection element such as a filter or balloon are becoming more common. A distal protection element provides one common means of controlling any emboli that may be created by a procedure. Guidewires may be highly specialized to provide quicker or easier access to particular intravascular sites and it may be difficult or expensive for a hospital or clinic to keep in stock all the guidewires a practitioner may desire to use. This problem (i.e. keeping a wide stock of guidewires on hand) is compounded with the introduction of new features such as distal protection to guidewires.

US 6,371,971 describes a filter system for entrapment of embolic material. The filter system includes a guidewire having a distal stop.

The present invention is defined by the features of the claims.

One embodiment of the invention pertains to a method of converting a non-distal protection guidewire into a distal protection guidewire. A desired guidewire is selected by the medical practitioner. A stopper is affixed to the guidewire at a desired location and a desired distal protection device is loaded onto the guidewire to create a distal protection guidewire. The stopper may be distal to the distal protection device or may be proximal. In one embodiment, the distal protection device is trapped between a distal stopper and a proximal stopper. In another embodiment, the stopper is integral with the distal protection device.

Another embodiment of the invention pertains to a kit for the conversion of a guidewire. The kit comprises a stopper having a first state where it has a larger lumen enabling it to be slid to a desired location on a guidewire and a second state where it has a smaller lumen and may be affixed to the guidewire. The stopper may be affixed by crimping or by adhesive. Another embodiment is a stopper made from a shape memory material that is affixed to a guidewire when it approaches body temperature. Another embodiment is made from a spring material that is biased to be in the second state.

The invention may be more completely understood in consideration of the following detailed description of various embodiments of the invention in connection with the accompanying drawings in which:
FIG. 1 is a partial plan view of a guidewire in a vascular lumen, the guidewire having a distal protection device and a stopper disposed thereon;
FIG. 2A is an end view of a stopper in a first state;
FIG. 2B is an end view of a stopper in a second state;
FIG. 3 is an orthogonal view of a stopper;
FIG. 4 is an orthogonal view of a stopper;
FIG. 5 is a partial plan view of a guidewire in a vascular lumen, the guidewire having a distal protection device and two stoppers disposed thereon; and
FIG. 6 is a partial plan view of a guidewire in a vascular lumen, the guidewire having a distal protection device having an integral stopper disposed thereon.

Reference is now made to the figures, in which like element numbers refer to like elements throughout.

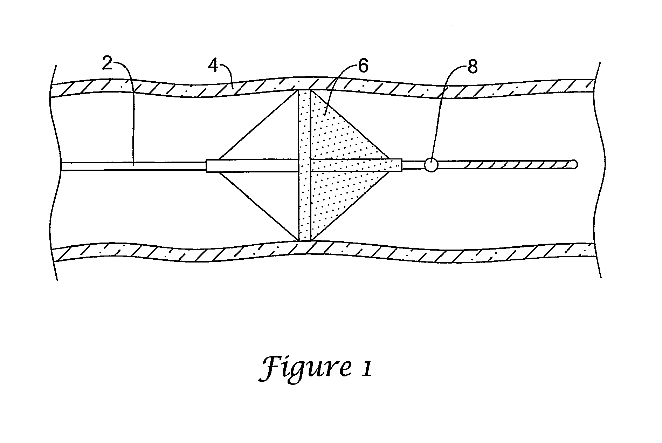

FIG. 1 is a plan view of an example guidewire 2 disposed in a vascular lumen defined by a vascular wall 4. Slideably disposed on guidewire 2 is a distal protection device 6. A stopper 8 is disposed distal of distal protection device 6 on guidewire 2. Stopper 8 prevents distal movement of distal protection device past the stopper. Thus a guidewire can be made into a distal protection guidewire. The term guidewire is herein merely a proxy for any elongate medical device. Thus a catheter, wire, or other elongate medical device may be suitable. Likewise, the term distal protection device is merely a proxy for any suitable device. The device merely needs a lumen through which the guidewire may be inserted.

This combination can be made by a medical practitioner at a hospital or clinic shortly prior to the therapeutic procedure in which the distal protection guidewire is used. Thus a distal protection guidewire can be customized by selecting a preferred guidewire, a preferred distal protection device and a preferred arrangement of the components (i.e. where the distal protection device is placed on the guidewire). Selective use of one or more stoppers 8 is used to make the conversion.

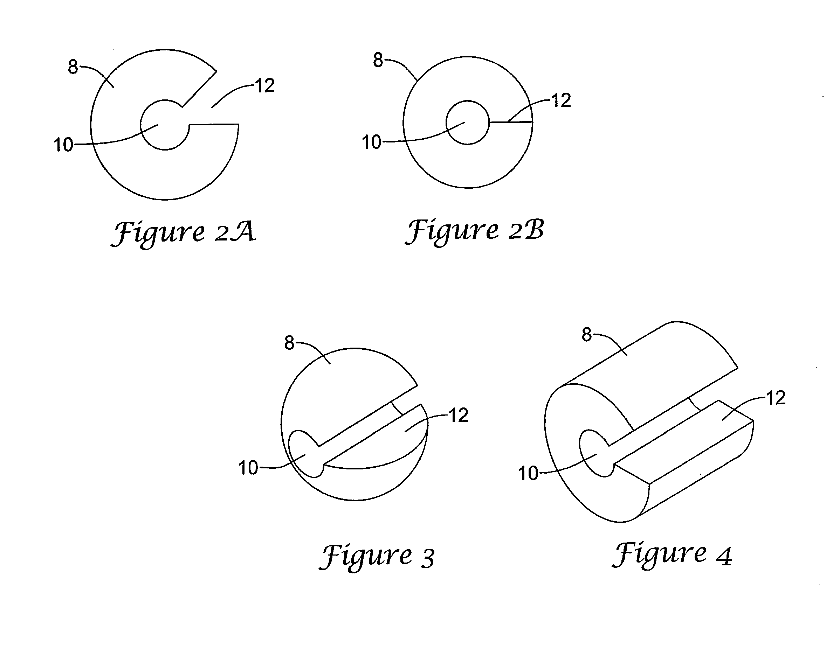

A stopper 8 has a longitudinal lumen 10 as can be seen in FIG. 2A, which is an end view of a stopper 8. In a first state, longitudinal lumen 10, which extends from a first end to a second end, has a first cross-sectional area that is sufficient to permit the stopper to slide over a selected guidewire to a selected location. In the first state, slit 12 defines a gap. Upon installation, the stopper is moved to a second state, where lumen 10 has a second cross-sectional area smaller than the first cross-sectional area of the first state. Slit 12 may be completely closed, as seen in FIG. 2B, or may merely define a smaller gap than in the first state. In other words, in the second state, the stopper may completely encircle the guidewire or may only partially do so. The stopper may have a spherical profile as shown in FIG. 3 or a cylindrical profile as shown in FIG. 4. The stopper may have other suitable profiles as well. Any profile which prevents a distal protection device from passing over it may be suitable. For example, the stopper may have a flared first end that tapers to a narrower second end. In the second state, stopper 8 fits tightly over a guidewire, fixing it to the guidewire. In some embodiments, the stopper has an interference fit with the guidewire and lumen 10 is completely occluded by the guidewire. In some embodiments, the inner surface of the stopper, which defines lumen 10, has a texture such as a plurality of ribs or wales that help to fix the stopper to the guidewire. In other embodiments, a pressure or heat sensitive adhesive may be used to help fix the stopper to the guidewire.

In one embodiment, the stopper is made from a plastically deformable material such as gold, silver, tin, or alloys thereof. Other such materials may be suitable. The term "plastically deformable" herein means that the stopper is made of such a material that, in moving from the first state to the second state, it will undergo plastic deformation. Such a stopper may be affixed to a guidewire by crimping. A crimping tool such as a pliers may be provided. The crimping tool may have jaws to receive the stopper and the jaws may have a pair of surfaces that have profiles that are inverse of the profile of the stopper. For example, if the stopper has a spherical profile, the jaws may include a pair of spherical indents sized and shaped to receive the stopper. The crimping tool may also include a stop to prevent over-crimping of the stopper. This stop may be a pair of opposing surfaces on the jaws or handles or may be any suitable stop. In some embodiments, a stopper may be packaged preloaded in the jaws of a suitable crimping tool.

In another embodiment, the stopper is a made from a shape memory material such as a nickel-titanium alloy. Other shape memory materials may also be suitable. The stopper moves to the first state when brought to body temperature. Body temperature herein means the normal range of temperatures found in the human body. The stopper moves to the second state when heated up from body temperature. In an alternative embodiment, the stopper may move to the second state when cooled down from body temperature. In some embodiments, the temperature at which the stopper moves to the second state is higher than body temperature, 65.6°C (150 degrees Fahrenheit) for example, but not so hot as to melt the polymers typically used in many guidewires. In one example installation, this stopper is heated to an installation temperature where it is in the first state and slid onto a guidewire, where it is allowed to cool down to the second state. The cooling may be hastened if desired by immersing the stopper in a sterile fluid or pouring a sterile fluid over the stopper.

In another example embodiment, the stopper is made from a spring material such as a spring steel that is biased towards the second state. The stopper is expanded to the first state, slid to a desired location on a selected guidewire and released to snap back to the second state. In one embodiment, the stopper is packaged in the second state, with a spacer holding slit 12 open. The spacer may have a handle thereon with which the stopper/spacer combination may be easily manipulated and with which the spacer can be easily removed from the stopper.

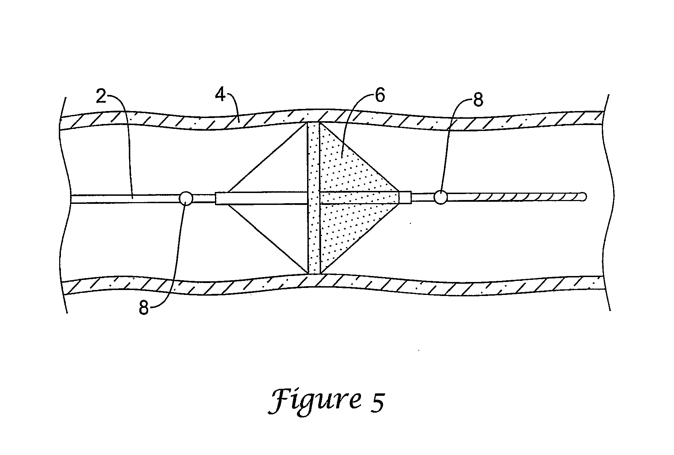

FIG. 5 is a plan view of a guidewire 2 in a vascular lumen defined by a vascular wall 4. Guidewire 2 has had two stoppers 8 attached to it, trapping a distal protection device 6 therebetween. Distal protection device 6 can rotate freely with respect to the guidewire but has limited proximal and distal movement.

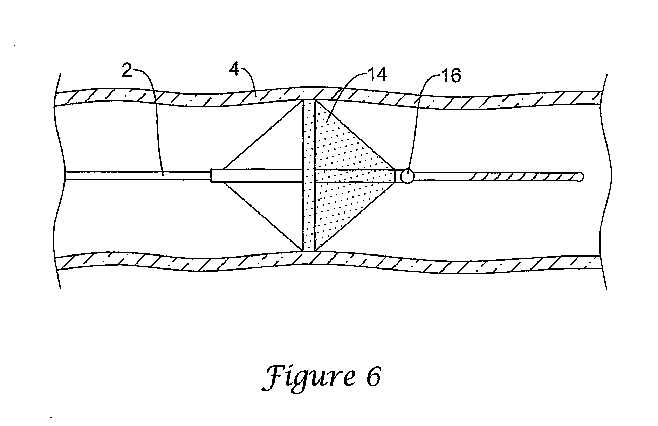

FIG. 6 is a plan view of a guidewire 2 in a vascular lumen defined by a vascular wall 4. A distal protection device 14 having an integrated stopper 16 is disposed on guidewire 2. Integrated stopper 16 may affix to guidewire 2 in substantially the same way as any of the stoppers 8. In some embodiments, the outer profile of stopper 16 may be significantly lower. Stopper 16 is depicted at the distal end of distal protection device 14 although in some embodiments the stopper may be located elsewhere on the distal protection device, such as in the middle or at the proximal end. Some embodiments may include more than one stopper 16.

In one example use, a guidewire is modified by a medical practitioner as described above and as shown in one of FIGs. 4, 5, or 6. The distal protection device may be confined in a catheter in a compact configuration. The guidewire and catheter are introduced concurrently percutaneously to a desired location such as the lumen of a blood vessel distal a treatment site. The catheter may then be removed to release the distal protection device. Other therapeutic devices such as an atherectomy device, stent deployment balloon or angioplasty balloon may then be introduced over the guidewire, if desired.

In another example use, a stopper 8 is affixed to a guidewire. The guidewire is then introduced percutaneously. A distal protection device is then introduced over the guidewire. The stopper 8 ensures the distal protection device won't slide off the distal end of the guidewire.

In another example use, a stopper 8 is affixed to a guidewire. An implantable filter such as a vena cava filter is loaded distal the stopper and is confined in a compact position by a catheter. The filter is introduced to a desired location and the guidewire may be used to push the filter distally from the end of the catheter or may be used to keep the filter from moving proximally as the catheter is withdrawn from over the filter. The catheter and guidewire are then withdrawn, leaving the filter installed.

To provide a medical practitioner with flexibility, stoppers 8 may be offered in various sizes to fit on guidewires of various outer diameters. Each stopper may be packaged in a separate sterile package. Installation tools may be manufactured inexpensively from a small number of parts such as molded plastic parts and each stopper may be packaged preloaded in an appropriate installation tool in a sterile package. The stoppers may also be packaged as a set with one or two stoppers packaged with an appropriate distal protection device. The packaging may include a spacer passing through the lumen of the stopper, which may provide easier handling of the stopper and may keep the stopper in the first state. The stopper may be easily slid off the spacer

Numerous advantages of the invention covered by this document have been set forth in the foregoing description. It will be understood, however, that this disclosure is, in many respects, only illustrative. Changes may be made in details, particularly in matters of shape, size, and arrangement of parts without exceeding the scope of the invention. The invention's scope is, of course, defined in the language in which the appended claims are expressed.

## Claims

1. A kit for the conversion of a guidewire into a distal protection guidewire, comprising:
a distal protection member (6) having a lumen therethrough for receiving any guidewire (2) having a outer diameter of a certain size or less; and
a stopper (8) fixable to a guidewire (2), the stopper (8) having a lumen (10) extending from a proximal end of the stopper (8) to a distal end of the stopper (8), **characterized in that** the stopper (8) has a first state where the lumen (10) has a first cross sectional area and a second state where the lumen (10) has a second cross-sectional area less than the first cross-sectional area, where the stopper (8) has an arcuate shape in the first state and a closed loop shape in the second state.

2. The kit of claim 1, wherein the lumen (10) of the stopper (8) is substantially empty.

3. The kit of claim 1, further comprising a second stopper fixable to a guidewire, the second stop having the same size as the first stopper.

4. The kit of claim 1, further comprising a second stopper fixable to a guidewire, the second stopper being fixable to guidewire of larger cross-sectional area than the first stopper.

5. The kit of claim 1, wherein the stopper (8) is plastically deformable from the first state to the second state.

6. The kit of claim 1, further comprising a tool for fixing the stopper (8) to a guidewire (2).

7. The kit of claim 6, wherein the tool has a pair of jaws having opposing surfaces whose profiles correspond to the profile of the outer surface of the stopper (8).

8. The kit of claim 7, wherein the tool has a stopper for preventing the jaws from closing beyond a certain point.

9. The kit of claim 8, wherein the stopper (8) is a pair of opposing surfaces on the jaws.

10. The kit of claim 1, wherein the stopper (8) is attached to the distal protection member (6).

11. A method of converting a guidewire into a distal protection guidewire, comprising the steps of:
providing a distal protection member having a distal end a proximal end and a lumen therebetween, the distal protection member having a first compact state and a second expanded state for trapping emboli; and
providing a first stopper defining an inner cross-sectional area, the stopper having a first state where the inner cross-sectional area has a first magnitude and a second state where the inner cross-sectional area has a second magnitude less that the first magnitude, the stopper having an outer profile in the second state such that the distal protection member cannot pass over it, where the stopper (8) has an arcuate shape in the first state and a closed loop shape in the second state.

12. The method of claim 11, wherein the stopper is compressible from the first state to the second state.

13. The method of claim 11, further comprising the step of providing a tool for fixing the stopper to a guidewire.

14. The method of claim 11, further comprising the step of providing a second stopper, the second stopper having a first state where the inner cross-sectional area has a first magnitude and a second state where the inner cross-sectional area has a second magnitude less that the first magnitude, the second stopper having an outer profile in the second state such that the distal protection member cannot pass over it.

15. The method of claim 11, wherein the step of providing the first stopper includes the step of providing the first stopper in the first state to a medical facility that performs therapeutic treatments of patients.

16. A percutaneous medical device, comprising
a stopper (8) having a first end, a second end and a lumen (10) therebetween,
the stopper (8) having first state where the lumen (10) has a first cross-sectional area and a second state where the lumen (10) has a second cross-sectional area less than the first cross-sectional area,
the stopper (8) having a slit (12) between the first end and the second end when in the first state, the slit (12) defining a gap,
the stopper (8) when in the second state having no bias to move to the first state at body temperature,
the stopper (8) having a distance of less than 3 cm between the first end and the second end; and
no member having a length of greater than 10 cm.

17. The device of claim 16, wherein the stopper (8) has an inner surface having a texture.

18. The device of claim 17, wherein the texture is a plurality of ribs disposed on the inner surface.

19. The device of claim 16, wherein the stopper (8) has a cylindrical profile in the second state.

20. The device of claim 16, wherein the stopper (8) has a spherical profile in the second state.

21. The device of claim 16, wherein the stopper (8) is plastically deformable.

22. The device of claim 21, wherein the stopper (8) includes a material selected from the group of tin, silver and gold.

23. The device of claim 22, wherein the stopper (8) comprises a shape memory material that biases the stopper to the second state at body temperature.

24. The device of claim 23, wherein the stopper (8) is biased to the first state at a temperature higher than body temperature.

25. The device of claim 16, wherein the stopper (8) is made of a material that is elastically deformable between the first state and the second state.

26. The device of claim 16, further comprising a sterile package surrounding the stopper (8).

27. The device of claim 26, wherein the lumen (10) is empty.

28. The device of claim 26, further comprising a spacer removably disposed in the lumen (10).

29. The device of claim 16, wherein the stopper (8) is affixable to a guidewire (2) by moving the stopper (8) to the second state when on the guidewire (2).

## Patentansprüche

1. Kit zur Umrüstung eines Führungsdrahts in einen Distalschutz-Führungsdraht, die aufweist:
ein Distalschutzteil (6) mit einem durchgehenden Lumen zum Aufnehmen jedes Führungsdrahts (2), der einen Außendurchmesser mit höchstens einer bestimmten Größe hat; und
einen Anschlag (8), der an einem Führungsdraht (2) befestigbar ist, wobei der Anschlag (8) ein Lumen (10) hat, das sich von einem proximalen Ende des Anschlags (8) zu einem distalen Ende des Anschlags (8) erstreckt, **dadurch gekennzeichnet, dass** der Anschlag (8) einen ersten Zustand, in dem das Lumen (10) eine erste Querschnittfläche hat, und einen zweiten Zustand hat, in dem das Lumen (10) eine zweite Querschnittfläche hat, die kleiner als die erste Querschnittfläche ist, wobei der Anschlag (8) eine Bogenform im ersten Zustand und eine geschlossene Schleifenform im zweiten Zustand hat.

2. Kit nach Anspruch 1, wobei das Lumen (10) des Anschlags (8) im Wesentlichen leer ist.

3. Kit nach Anspruch 1, ferner mit einem zweiten Anschlag, der an einem Führungsdraht befestigbar ist, wobei der zweite Anschlag die gleiche Größe wie der erste Anschlag hat.

4. Kit nach Anspruch 1, ferner mit einem zweiten Anschlag, der an einem Führungsdraht befestigbar ist, wobei der zweite Anschlag an Führungsdraht mit größerer Querschnittfläche als der erste Anschlag befestigbar ist.

5. Kit nach Anspruch 1, wobei der Anschlag (8) aus dem ersten Zustand in den zweiten Zustand plastisch verformbar ist.

6. Kit nach Anspruch 1, ferner mit einem Werkzeug zum Befestigen des Anschlags (8) an einem Führungsdraht (2).

7. Kit nach Anspruch 6, wobei das Werkzeug ein Paar Backen mit gegenüberliegenden Oberflächen hat, deren Profile dem Profil der Außenfläche des Anschlags (8) entsprechen.

8. Kit nach Anspruch 7, wobei das Werkzeug einen Anschlag zum Verhindern hat, dass sich die Backen über einen bestimmten Punkt hinaus schließen.

9. Kit nach Anspruch 8, wobei der Anschlag (8) ein Paar gegenüberliegende Oberflächen auf den Backen ist.

10. Kit nach Anspruch 1, wobei der Anschlag (8) am Distalschutzteil (6) angebracht ist.

11. Verfahren zum Umrüsten eines Führungsdrahts in einen Distalschutz-Führungsdraht mit den Schritten:
Bereitstellen eines Distalschutzteils mit einem distalen Ende und einem proximalen Ende sowie einem Lumen dazwischen, wobei das Distalschutzteil einen ersten kompakten Zustand und einen zweiten expandierten Zustand zum Einfangen von Emboli hat; und
Bereitstellen eines ersten Anschlags, der eine Innenquerschnittfläche abgrenzt, wobei der Anschlag einen ersten Zustand, in dem die Innenquerschnittfläche eine erste Größe hat, und einen zweiten Zustand hat, in dem die Innenquerschnittfläche eine zweite Größe hat, die kleiner als die erste Größe ist, und der Anschlag ein Außenprofil im zweiten Zustand so hat, dass das Distalschutzteil nicht darüber laufen kann, wobei der Anschlag (8) eine Bogenform im ersten Zustand und eine geschlossene Schleifenform im zweiten Zustand hat.

12. Verfahren nach Anspruch 11, wobei der Anschlag aus dem ersten Zustand in den zweiten Zustand komprimierbar ist.

13. Verfahren nach Anspruch 11, ferner mit dem Schritt des Bereitstellens eines Werkzeugs zum Befestigen des Anschlags an einem Führungsdraht.

14. Verfahren nach Anspruch 11, ferner mit dem Schritt des Bereitstellens eines zweiten Anschlags, wobei der zweite Anschlag einen ersten Zustand, in dem die Innenquerschnittfläche eine erste Größe hat, und einen zweiten Zustand hat, in dem die Innenquerschnittfläche eine zweite Größe hat, die kleiner als die erste Größe ist, und der zweite Anschlag ein Außenprofil im zweiten Zustand so hat, dass das Distalschutzteil nicht darüber laufen kann.

15. Verfahren nach Anspruch 11, wobei der Schritt des Bereitstellens des ersten Anschlags den Schritt des Bereitstellens des ersten Anschlags im ersten Zustand für eine medizinische Einrichtung aufweist, die therapeutische Behandlungen von Patienten durchführt.

16. Perkutanes Medizinprodukt, das aufweist:
ein Anschlag (8) mit einem ersten Ende, einem zweiten Ende und einem Lumen (10) dazwischen,
wobei der Anschlag (8) einen ersten Zustand, in dem das Lumen (10) eine erste Querschnittfläche hat, und einen zweiten Zustand hat, in dem das Lumen (10) eine zweite Querschnittfläche hat, die kleiner als die erste Querschnittfläche ist,
der Anschlag (8) einen Schlitz (12) zwischen dem ersten Ende und dem zweiten Ende im ersten Zustand hat und der Schlitz (12) einen Spalt abgrenzt,
der Anschlag (8) im zweiten Zustand keine Vorspannung hat, um sich bei Körpertemperatur in den ersten Zustand zu bewegen,
der Anschlag (8) einen Abstand von weniger als 3 cm zwischen dem ersten Ende und dem zweiten Ende hat; und
kein Teil eine Länge von mehr als 10 cm hat.

17. Produkt nach Anspruch 16, wobei der Anschlag (8) eine Innenfläche mit einer Textur hat.

18. Produkt nach Anspruch 17, wobei es sich bei der Textur um mehrere Rippen handelt, die auf der Innenfläche angeordnet sind.

19. Produkt nach Anspruch 16, wobei der Anschlag (8) ein zylindrisches Profil im zweiten Zustand hat.

20. Produkt nach Anspruch 16, wobei der Anschlag (8) ein kugelförmiges Profil im zweiten Zustand hat.

21. Produkt nach Anspruch 16, wobei der Anschlag (8) plastisch verformbar ist.

22. Produkt nach Anspruch 21, wobei der Anschlag (8) ein Material aufweist, das aus der Gruppe aus Zinn, Silber und Gold ausgewählt ist.

23. Produkt nach Anspruch 22, wobei der Anschlag (8) ein Formgedächtnismaterial aufweist, das den Anschlag bei Körpertemperatur in den zweiten Zustand vorspannt.

24. Produkt nach Anspruch 23, wobei der Anschlag (8) bei einer höheren Temperatur als Körpertemperatur in den ersten Zustand vorgespannt ist.

25. Produkt nach Anspruch 16, wobei der Anschlag (8) aus einem Material hergestellt ist, das zwischen dem ersten Zustand und dem zweiten Zustand elastisch verformbar ist.

26. Produkt nach Anspruch 16, ferner mit einer sterilen Verpackung, die den Anschlag (8) umgibt.

27. Produkt nach Anspruch 26, wobei das Lumen (10) leer ist.

28. Produkt nach Anspruch 26, ferner mit einem Abstandshalter, der im Lumen (10) entfernbar angeordnet ist.

29. Produkt nach Anspruch 16, wobei der Anschlag (8) an einem Führungsdraht (2) fixierbar ist, indem der Anschlag (8) in den zweiten Zustand bewegt wird, wenn er sich auf dem Führungsdraht (2) befindet.

## Revendications

1. Kit pour la conversion d'un fil-guide en fil-guide à protection distale, comprenant :
un élément de protection distale (6) avec une lumière intérieure pour la réception de tout fil-guide (2) ayant un diamètre extérieur inférieur ou égal à une grandeur définie ; et
un élément d'arrêt (8) fixable sur un fil-guide (2), ledit élément d'arrêt (8) ayant une lumière (10) s'étendant d'une extrémité proximale de l'élément d'arrêt (8) à une extrémité distale de l'élément d'arrêt (8), **caractérisé en ce que** l'élément d'arrêt (8) présente un premier état où la lumière (10) a une première surface de section transversale et un deuxième état où la lumière (10) a une deuxième surface de section transversale inférieure à la première surface de section transversale, l'élément d'arrêt (8) présentant une forme en arc dans le premier état et une forme en boucle fermée dans le deuxième état.

2. Kit selon la revendication 1, où la lumière (10) de l'élément d'arrêt (8) est sensiblement vide.

3. Kit selon la revendication 1, comprenant en outre un deuxième élément d'arrêt fixable sur un fil-guide, ledit deuxième élément d'arrêt étant de même dimension que le premier élément d'arrêt.

4. Kit selon la revendication 1, comprenant en outre un deuxième élément d'arrêt fixable sur un fil-guide, ledit deuxième élément d'arrêt étant fixable sur un fil-guide de surface de section transversale supérieure à celle du premier élément d'arrêt.

5. Kit selon la revendication 1, où l'élément d'arrêt (8) est plastiquement déformable du premier état au deuxième état.

6. Kit selon la revendication 1, comprenant en outre un outil pour fixer l'élément d'arrêt (8) sur un fil-guide (2).

7. Kit selon la revendication 6, où l'outil présente une paire de mâchoires avec des surfaces opposées dont les profils correspondent au profil de la surface extérieure de l'élément d'arrêt (8).

8. Kit selon la revendication 7, où l'outil présente un élément d'arrêt pour empêcher les mâchoires de se fermer au-delà d'un point défini.

9. Kit selon la revendication 8, où l'élément d'arrêt (8) est une paire de surfaces opposées sur les mâchoires.

10. Kit selon la revendication 1, où l'élément d'arrêt (8) est fixé à l'élément de protection distale (6).

11. Procédé de conversion d'un fil-guide en fil-guide à protection distale, comprenant les étapes de :
préparation d'un élément de protection distale avec une extrémité distale, une extrémité proximale et une lumière entre celles-ci, l'élément de protection distale présentant un premier état compact et un deuxième état d'expansion pour la protection contre les embolies ; et de
préparation d'un premier élément d'arrêt définissant une surface intérieure de section transversale, l'élément d'arrêt présentant un premier état où la surface intérieure de section transversale a une première grandeur et un deuxième état où la surface intérieure de section transversale a une deuxième grandeur inférieure à la première grandeur,
l'élément d'arrêt présentant dans le deuxième état un profil extérieur empêchant son dépassement par l'élément de protection distale, l'élément d'arrêt (8) présentant une forme en arc dans le premier état et une forme en boucle fermée dans le deuxième état.

12. Procédé selon la revendication 11, où l'élément d'arrêt est compressible du premier état au deuxième état.

13. Procédé selon la revendication 11, comprenant en outre l'étape de préparation d'un outil pour fixer l'élément d'arrêt sur un fil-guide.

14. Procédé selon la revendication 11, comprenant en outre l'étape de préparation d'un deuxième élément d'arrêt, le deuxième élément d'arrêt présentant un premier état où la surface intérieure de section transversale a une première grandeur et un deuxième état où la surface intérieure de section transversale a une deuxième grandeur inférieure à la première grandeur, le deuxième élément d'arrêt présentant dans le deuxième état un profil extérieur empêchant son dépassement par l'élément de protection distale.

15. Procédé selon la revendication 11, où l'étape de préparation du premier élément d'arrêt comprend l'étape de délivrance du premier élément d'arrêt dans le premier état à une installation médicale administrant des traitements thérapeutiques aux patients.

16. Dispositif médical percutané, comprenant :
un élément d'arrêt (8) avec une première extrémité, une deuxième extrémité et une lumière (10) entre celles-ci,
l'élément d'arrêt (8) présentant un premier état où la lumière (10) a une première surface de section transversale et un deuxième état où la lumière (10) a une deuxième surface de section transversale inférieure à la première surface de section transversale,
l'élément d'arrêt (8) présentant une fente (12) entre la première extrémité et la deuxième extrémité dans le premier état, la fente (12) définissant un interstice,
l'élément d'arrêt (8) dans le deuxième état n'étant pas contraint à se déplacer vers le premier état à température corporelle,
l'élément d'arrêt (8) présentant une distance inférieure à 3 cm entre la première extrémité et la deuxième extrémité ; et
aucun composant ne présentant une longueur supérieure à 10 cm.

17. Dispositif selon la revendication 16, où l'élément d'arrêt (8) a une surface intérieure pourvue d'une texture.

18. Dispositif selon la revendication 17, où la texture est une pluralité de nervures disposées sur la surface intérieure.

19. Dispositif selon la revendication 16, où l'élément d'arrêt (8) a un profil cylindrique dans le deuxième état.

20. Dispositif selon la revendication 16, où l'élément d'arrêt (8) a un profil sphérique dans le deuxième état.

21. Dispositif selon la revendication 16, où l'élément d'arrêt (8) est plastiquement déformable.

22. Dispositif selon la revendication 21, où l'élément d'arrêt (8) comprend un matériau sélectionné dans le groupe composé d'étain, d'argent et d'or.

23. Dispositif selon la revendication 22, où l'élément d'arrêt (8) comprend un matériau à mémoire de forme qui contraint l'élément d'arrêt dans le deuxième état à température corporelle.

24. Dispositif selon la revendication 23, où l'élément d'arrêt (8) est contraint dans le premier état à température supérieure à la température corporelle.

25. Dispositif selon la revendication 16, où l'élément d'arrêt (8) est en matériau élastiquement déformable entre le premier état et le deuxième état.

26. Dispositif selon la revendication 16, comprenant en outre un emballage stérile entourant l'élément d'arrêt (8).

27. Dispositif selon la revendication 26, où la lumière (10) est vide.

28. Dispositif selon la revendication 26, comprenant en outre un élément d'espacement disposé de manière amovible dans la lumière (10).

29. Dispositif selon la revendication 16, où l'élément d'arrêt (8) peut être fixé sur un fil-guide (2) par déplacement de l'élément d'arrêt (8) vers le deuxième état quand il est sur le fil-guide (2).
